# EUROPÄISCHE PATENTSCHRIFT

(11) **EP 3 714 841 B1**
(45) Veröffentlichungstag und Bekanntmachung des Hinweises auf die Patenterteilung: **16.03.2022**
(21) Anmeldenummer: 19164795.7
(22) Anmeldetag: 25.03.2019
(51) Int. Cl.: A61F 2/36

(54) **KURZSCHAFTPROTHESE**
SHORT SHAFT PROSTHETIC
PROTHÈSE À TIGE COURTE

(43) Veröffentlichungstag der Anmeldung: 30.09.2020
(73) Patentinhaber: Justus-Liebig-Universität Gießen, 35390 Gießen (DE)
(72) Erfinder: Jahnke, Alexander, 35392 Gießen (DE); Harz, Torben, 35394 Gießen (DE); Stiller, Alexander Martin, 35390 Gießen (DE)
(74) Vertreter: Stumpf, Peter

(56) Entgegenhaltungen:
- DE-A1- 3 609 119
- DE-T2- 69 630 102
- US-A- 5 507 832
- US-A1- 2007 219 641

## Beschreibung

### Kurzschaftprothese

Die vorliegende Erfindung betrifft eine neuartige Kurzschaftprothese zur Verwendung als Hüftendoprothese.

### Allgemeines Gebiet der Erfindung

Das Hüftgelenk ist nach dem Kniegelenk das größte Gelenk des menschlichen Körpers. Es ist ein Kugelgelenk, das durch kräftige Bänder und die Bein- und Beckenmuskulatur verstärkt wird. Seine Funktion ist entscheidend für den aufrechten Gang und die freie Beweglichkeit des gleichseitigen Beines. Im Laufe des Lebens kann es zu einem natürlichen Verschleiß des Gelenkknorpels kommen, der so ausgeprägt ist, dass es zu starken Schmerzen bei der Belastung und einer weitgehenden Einschränkung der Gelenkbeweglichkeit kommt.

Deshalb zählt zu den häufigsten Operationen in der orthopädischen Chirurgie aktuell die Implantation von Hüftendoprothesen. Jedes Jahr werden allein in Deutschland über 230.000 primäre Hüftendoprothesen implantiert, eine Anzahl, die analog zu dem Anstieg der immer älter werdenden Bevölkerung, in den letzten Jahrzehnten stetig angestiegen ist. Durch den operativen Einsatz eines künstlichen Gelenks kann den Patienten postoperativ eine sofortige schmerzfreie Mobilisation gewährleistet werden. Hierbei muss allerdings berücksichtigt werden, dass gerade Patienten mit einer sekundären Arthrose meist zu den jüngeren Patienten gehören.

Hüftendoprothesen unterliegen dabei verschiedenen mechanischen Herausforderungen. So fällt die durch das Gewicht eines Patienten auf das Hüftgelenk wirkende Kraft nicht mit der Schenkelhals- und der Diaphysenachse des Femurs zusammen, so dass ein Drehmoment im Varussinn auf das Femur wirkt. Schenkelhals und Diaphyse erfahren dabei in der Frontalebene eine Biegebeanspruchung. Im medialen Anteil, also im Bereich des Calcar femoris, treten Druckspannungen auf, während im lateralen Anteil, also im Bereich des Trochanter majors, kleinere Zugspannungen herrschen im Vergleich zur Druckspannung. Die Hauptmasse des Knochengewebes liegt dementsprechend in diesen Bereichen, was durch ein gerichtetes trabekuläres System gekennzeichnet ist.

Der Ersatz des Hüftgelenks durch eine zementfreie Hüftendoprothese verändert diesen Beanspruchungsmechanismus. Das Varusdrehmoment, das nun über den Prothesenschaft in das Femur eingeleitet wird, muss von einem Querkräftepaar kompensiert werden. Während des Gehens erzeugt die resultierende Gelenkkraft ein Varusdrehmoment. Dieses Moment presst den proximalen Anteil der Prothese gegen die mediale Kortikalis und den distalen Anteil gegen die laterale Kortikalis. Im Bereich der Querkräfte entstehen erhöhte Spannungskonzentrationen. Je nach Länge variieren Drehpunkt und Hebelarme des Prothesenschafts.

In den letzten Jahren gab es Neuentwicklungen in der Hüftendoprothetik, um die Beeinträchtigungen im natürlichen Beanspruchungsmechanismus und den resultierenden Knochen-Umbauprozessen entgegenzuwirken. Im Vordergrund stand dabei, durch das Implantat so wenig wie möglich in die physiologische Spannungsverteilung des Femurs einzugreifen.

Inzwischen scheint sich in diesem Zusammenhang die zementfreie Versorgung mit kurzen, den Schenkelhals zumindest partiell erhaltenden Prothesen immer stärker durchzusetzen. Die Theorie hinter diesen Kurzschaftprothesen ist eine möglichst proximale Verankerung, wobei unter Belassung des trabekulären Systems das Varusdrehmoment entlang der physiologischen Spannungslinien des Calcars femoralis und des lateralen Femurs nach distal in die Diaphyse geleitet werden soll. Dokument US 5 507 832 A offenbart eine Kurzschaftprothese für ein künstliches Hüftgelenk gemäß dem Oberbegriff von Anspruch 1.

### Aufgabe

Aufgabe der vorliegenden Erfindung ist es eine Kurzschaftprothese zur Verwendung als Hüftendoprothese bereitzustellen, welche sich elastisch deutlich besser verformt und so den Erhalt der Spongiosastruktur optimiert.

### Lösung der Aufgabe

Gelöst wird diese Aufgabe durch die kennzeichnenden Merkmale des Anspruchs 1. Weitere vorteilhafte Ausgestaltungen ergeben sich aus den Unteransprüchen. Wichtige Begriffe:
Proximal: Proximal bedeutet "näher" zur Körpermitte hin oder "näher" gelegen, auch: dem zentralen Teil eines Körpergliedes bzw. der Körpermitte zu gelegen und bezieht sich hier auf das protheseneigene Koordinatensystem.

Distal: Distal bedeutet "weiter" von der Körpermitte "entfernt" gelegen, auch: Bezeichnung für weiter vom Mittelpunkt oder der Mittelachse eines Körpers oder charakteristischer Bezugspunkte entfernt liegender Teile und bezieht sich das protheseneigene Koordinatensystem.

Die erfindungsgemäße Kurzschaftprothese umfasst einen Schaft 8, wobei dieser einen distalen Bereich 12 und einen proximalen Bereich 11 umfasst, wobei der distale Bereich 12 wenigstens 50% der Gesamtlänge des Schafts 8 umfasst. Weiterhin umfasst die erfindungsgemäße Kurzschaftprothese eine mit dem Schaft 8 verbundene proximale Prothesenschulter 13, welche so ausgebildet ist, dass sie mit einem Konus 14 zur Aufnahme des Prothesenkopfes 1 verbindbar ist. Die Verbindung kann dabei über eine Hülse (z.B. eine Geradehülse oder eine Schwenkhülse) erfolgen. Der Schaft 8 weist eine nach distal verjüngende Form auf. Das heißt, der Querschnitt in horizontaler Ebene der Prothese erfährt eine stetige Abnahme nach distal. Je nach Prothesengröße und Flankensteigung variieren die Abmaße der Prothese selbstverständlich.

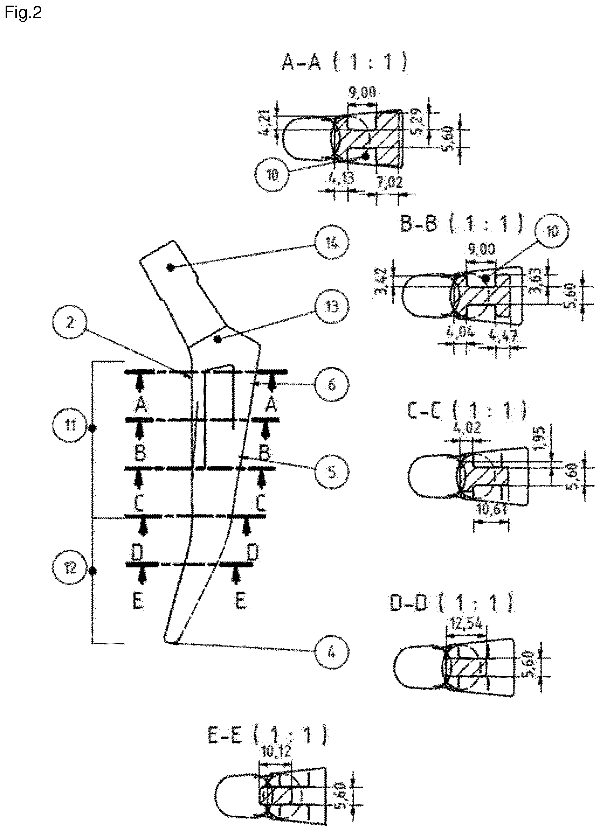

Dabei besteht der Schaft 8 aus einer medialen Flanke 2, einer lateralen Flanke 6 und einem Steg 5 zwischen diesen Flanken 2 und 6. Im proximalen Bereich 11 der hier dargestellten Prothesengröße (siehe Schnittansicht A-A, Fig.2), an der eine erste Differenzierung von Steg und Flanken vorgenommen werden kann, beträgt in einer beispielhaften Ausführungsform die kombinierte Gesamtbreite der Prothesenflanken 11,15 mm, wobei die laterale Flanke hierbei eine Breite von 7,02 mm und die mediale Flanke eine Breite von 4,13 mm aufweist. Die Flankentiefen betragen in diesem Beispiel lateral 5,29 mm und medial 4,21 mm. Die Werte für die Breite des Steges können je nach Patient individuell verschieden sein, sodass sich hier keine allgemeingültigen Werte für die absoluten Größen für Tiefe und Breite der einzelnen Abschnitte des Schaftes 8 angeben lassen. Weiterhin unterliegen diese Werte im Verlauf von proximal nach distal einer fortwährenden Veränderung und sind abhängig von der eingesetzten Prothesengröße.

Ein wichtiges Merkmal eines mechanischen Systems ist die sogenannte neutrale Faser. Als neutrale Faser bezeichnet man in der Festigkeitslehre diejenige Faser oder Schicht eines Balkenquerschnitts, deren Länge sich bei Verdrehen bzw. Biegen nicht ändert. Dort verursacht die Beanspruchung keine Zug- oder DruckSpannung. Die neutrale Faser 10 der erfindungsgemäßen Kurzschaftprothese bezeichnet dabei derjenige Bereich des Schaftprofils der Prothese, deren Länge sich bei Verdrehen bzw. Biegen nicht ändert.

Im proximalen Bereich 11 der Prothese, an der eine erste sinnvolle Differenzierung von Steg und Flanke vorgenommen werden kann, beträgt in der oben genannten beispielhaften Ausführungsform die Gesamtbreite des Stegs 5, der beide Flanken verbindet, 9,00 mm und eine Tiefe von 5,6 mm. Die Tiefe des Stegs ist dabei über die gesamte Länge des Steges gleichbleibend. Die Flanken verjüngen sich von proximal und laufen auf das Stegniveau aus. Das Breitenverhältnis des Steges zu den Flanken liegt im proximalen Bereich 11 bei wenigstens 0,8, wodurch er ein Doppel-T-Trägerquerschnitt im proximalen Bereich 11 aufweist. Dadurch wird eine Aussparung der neutralen Faser 10 im Verlauf zur distalen Prothesenspitze 9 hin erreicht, wodurch sich die erfindungsgemäße Kurzschaftprothese elastisch verformen kann und so den Erhalt der Spongiosastruktur optimiert.

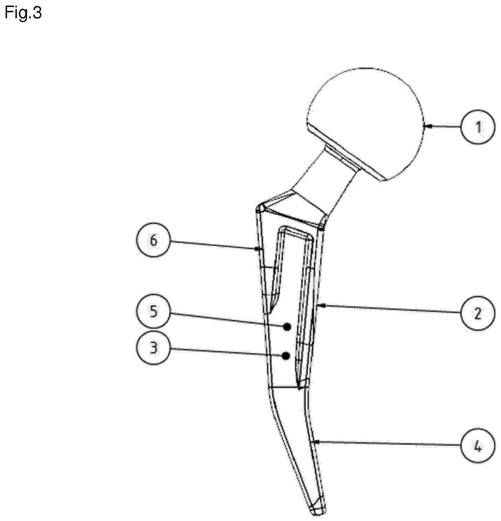

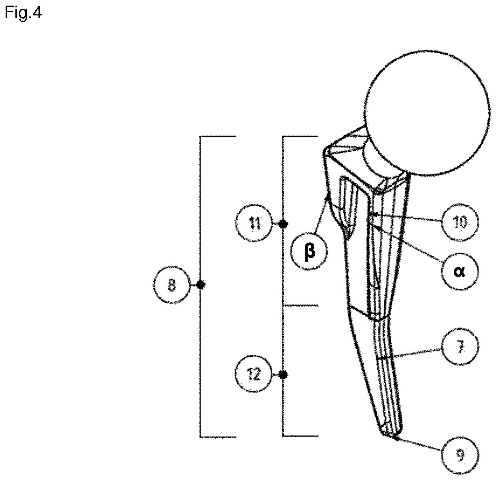

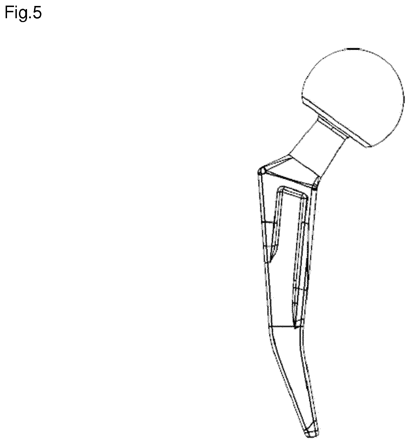

Das Grundprinzip des Schaftdesigns wird auch anhand der vorliegenden Zeichnungen Fig.2 bis Fig.5 beschrieben. Eine genaue Spezifikation der Abmaße ist aufgrund unterschiedlicher Schaftgrößen nicht generell festlegbar. Die genannten absoluten Größen sind damit beispielhaft zu verstehen und können für bestimmte Prothesen auch abweichen. Das Größenverhältnis der Steges 5 und der Flanken 2 und 6 bleibt jedoch gleich.

Abbildung 2 zeigt einen Schnittverlauf in verschiedenen Ebenen der Prothese. Sie zeigt die kontinuierliche Veränderung der Flankenbreiten und -tiefen, sowie die ortsabhängige Breitenänderung des Steges. Im distalen Schaftverlauf weist der Prothesenschaft einen rechteckigen Querschnitt auf, der sich ebenfalls nach distal verjüngt. Der distale Bereich 12 des Schaftes ist vorzugsweise ca. 15° nach medial gekrümmt. Alle Kanten des Schaftes 8 sind abgerundet, sodass keine scharfen Kanten existieren. Dies ist in Fig.2 im Bereich von Schnittansicht D-D bis zur Prothesenspitze 9 dargestellt.

Vorzugsweise ist der Schaft 8 so ausgebildet, dass die erfindungsgemäße Kurzschaftprothese zementfrei implantiert werden kann. Realisiert wird dies durch eine biokompatible Beschichtung 3 im proximalen Schaftbereich 11. Definiert wird die Biokompatibilität von medizinischen Werkstoffen in der ISO 10993 1-20. Diese Art von Kurzschaftprothesen werden auch als zementfreie Kurzschaftprothesen bezeichnet. Zementfreie Hüftendprothesen sind die heutzutage medizinisch bevorzugte Prothesenvariante, da diese eine stabilere Verbindung mit dem Knochen ermöglichen. Dies ist jedoch nicht immer möglich.

Die biokompatible Beschichtung 3 im proximalen Schaftbereich 11 kann dabei beispielsweise aus Dicalciumphosphat oder Hydroxylapathit bestehen. Diese Materialien gelten als besonders biokompatibel und sind geeignet den mechanischen Anforderungen bei einer Verwendung als Hüftendprothese über einen langen Zeitraum Stand zu halten. Die biokompatible Beschichtung 3 kann dabei auch bioaktiv ausgeführt sein. In der Endoprothetik versteht man unter bioaktiv die Reaktion eines Knochens auf das Implantat, welche eine Adhäsion vom Knochen an der Grenzschicht zum Implantat zulässt. Typische Werkstoffe, welche meist zu dem Begriff bioaktiv gezählt werden, bestehen aus Kohlenstoffen, Keramiken und Gläsern (beispielsweise BioGlass^{®}).

Alternativ kann ein zementfreier Schaft 8 auch mit eine Reintitanbeschichtung aufweisen. Beschichtungen, auch Reintitanbeschichtungen sind grundsätzlich auf jeder Prothesenart, hinsichtlich des verwendeten Werkstoffes, realisierbar.

Die Mittenrauigkeit des zementfrei implantierbaren Schaftes 8 liegt idealerweise bei ca. Ra = 340 µm, um eine sichere Verankerung zu garantieren.

Vorzugsweise besteht die erfindungsgemäße Kurzschaftprothese aus einer Titan- und/oder Titanoxid-Legierung.

Alternativ besteht der Schaft aus einer Cobalt-Chrom-Molybdän-Legierung. Dabei weist er eine besonders glatte Oberfläche mit einer Mittenrauigkeit von ca. 0,4 µm auf. Diese Ausführungsform ist besonders bei zementierten Implantation bevorzugt.

Die bestehenden Flanken im proximalen Schaftbereich ermöglichen sowohl eine sofortige Torsionsstabilität als auch eine breitflächige Anlage im medialen als auch lateralen Femurkortex. Der auslaufende Steg 5 der Prothese, der die beiden Flanken im oberen Schaftbereich verbindet, weist im distalen Schaftverlauf unterhalb der Flanken eine blank polierte unbeschichtete Oberfläche 7 auf. Blank poliert heißt hierbei, dass die Mittenrauigkeit Ra besonders gering ist. Sie beträgt unter 1 µm. Die Mittenrauigkeit der Oberfläche 7 liegt idealerweise bei ca. Ra = 0,4 µm und dient lediglich der distalen lateralen Anlage als auch der intramedullären Führung.

Der distale Prothesenschaft der erfindungsgemäßen Kurzschaftprothese ermöglicht dabei keine Verankerungsmöglichkeit im distalen Schaftbereich 12, sondern dient als laterale Anlage um den Schaft 8 über einen verlängerten Hebelarm in eine elastische Verformung unter Biegebelastung zwingen.

Durch die Aussparung der neutralen Faser 10, wird die elastische Verformung der Prothese bei Biegung und Torsion bei gleichzeitig maximierter Torsionsstabilität durch das Doppel-T-Träger-Profil heraufgesetzt; die nach proximal größer werdenden Flanken sorgen zudem für eine Verblockung der Prothese im proximalen Femurbereich und sorgen somit für eine physiologischere Lasteinleitung.

Die mediale Flanke 2 weist idealerweise einen Steigungswinkel **α** von wenigstens 3° auf. Die laterale Flanke 6 weißt idealerweise einen Steigungswinkel **β** von wenigstens 3,2° auf. Dabei laufen beide Flanken im distalen Bereich mit einer Rundung auf den Schaftsteg 5 aus.

Die mediale Flanke 2 als auch die laterale Flanke 6 können dabei auch unterschiedliche Steigungswinkel aufweisen, um so der individuellen Anatomie und Femurkonfiguration des Patienten intraoperativ entsprechen zu können.

Zur Implantation der erfindungsgemäßen Kurzschaftprothese werden vorzugsweise speziell auf die erfindungsgemäße Kurzschaftprothese abgestimmte Stegraspeln und Flankenraspeln vorgeschlagen.

Die Stegraspel weist eine Raspelstruktur in den proximalen zwei Dritteln auf und ist im distalen Raspelbereich poliert.

Sie weist eine doppelkonische nach distal verjüngende Form auf, welche dem Stegprofil der finalen Prothese nachempfunden ist.

Die Flankenraspeln weisen die Doppel-T-Träger-Struktur der erfindungsgemäßen Kurzschaftprothese auf und werden sukzessive von klein nach groß eingesetzt. Im Gegensatz zu der Stegraspel weisen die Flankenraspeln eine glatt polierte Oberflächenstruktur des Steges auf. Lediglich die Flanken weisen eine Raspelstruktur auf. Der blank polierte Steg kann so lediglich als Führung der Flankenraspel im bereits präpariertem Merkraumkanal fungieren, ohne diesen weiter aufzuraspeln.

Zur Implantation der erfindungsgemäßen Kurzschaftprothese mittels Stegraspel und Flankenraspel eignet sich beispielsweise das im Folgenden beschriebene Verfahren:
In einem ersten Schritt I wird mit der Stegraspel der Markraum nach der Osteotomie des Femurhalses sukzessive von klein nach groß aufgeraspelt, bis eine laterale Anlage der Stegraspelspitze am Femurkortex erfolgt und die Stegraspel mittig innerhalb der Osteotomie unter Berücksichtigung der notwendigen Antetorsionsstellung des Femurs lokalisiert ist.

Die Positionierung und die Größe der Stegraspel in Schritt I werden anschließend in einem Schritt II intraoperativ durch eine anterior-posteriore Röntgenkontrolle durch den Operateur überprüft.

Wenn in Schritt II eine adäquate Schaftstellung der Stegraspel festgestellt wurde, wird diese in einem Schritt III aus dem Markraum entfernt.

Anschließend wird in einem Schritt IV mit einer Flankenraspel das Doppel-T-Träger Profil der Prothese im proximalen Bereich 11 sukzessive aufgeraspelt bis die erfindungsgemäße Kurzschaftprothese eingefügt werden kann. Hierbei kann der Operateur entsprechend der individuellen anatomischen Gegebenheiten verschiedene Flankenraspeln mit unterschiedlichen Flankensteigungen einsetzen, um einen möglichst kortikalen Kontakt der Prothesenflanken im proximalen Bereich 11 bei gleichzeitig maximalen Erhalt der Spongiosastruktur zu realisieren.

Nach dem Aufraspeln der Flanken wird die finale Flankenraspel wiederum im Markraum belassen und in einem Schritt V eine Röntgenkontrolle sowie eine Probereposition des Gelenks durchgeführt.

Nach positiver Bewertung der Schaftstellung und der Probereposition wird in einem Schritt VI die erfindungsgemäße Kurzschaftprothese in das durch die Schritte I bis IV freigeraspelte Implantatlager eingeschlagen bzw. einzementiert. Bei der zementfreien Variante geschieht dies über ein Fügeverfahren vorzugsweise mit einem Hammer.

### Abbildungslegenden und Bezugszeichenliste

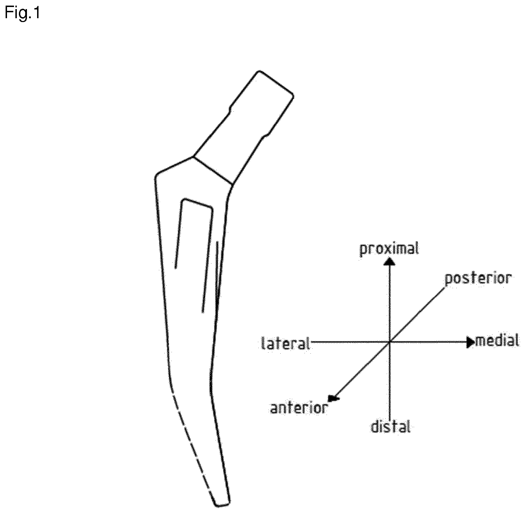

Fig.1 Prinzipabbildung eines Schafts der erfindungsgemäßen Kurzschaftprothese mit Richtungsbezeichnungen
Fig. 2: Schnittansichten eines Schafts der erfindungsgemäßen Kurzschaftprothese
Fig. 3: Darstellung der erfindungsgemäßen Kurzschaftprothese als zementfreie Variante mit einer Beschichtung 3 und gefügtem Prothesenkopf
Fig. 4: Darstellung der erfindungsgemäßen Kurzschaftprothese mit einer Darstellung des proximalen Bereiches 11 des Schafts 8 und des distaler Bereich des Schafts 12
Fig. 5: Darstellung der erfindungsgemäßen Kurzschaftprothese als zementierte Variante mit einer unbeschichteten Oberfläche

### Bezugszeichenliste

- 1: Prothesenkopf
- 2: mediale Flanke
- 3: Beschichtung
- 4: Schaftspitze
- 5: Steg
- 6: laterale Flanke
- 7: unbeschichtete Oberfläche des Schafts
- 8: Schaft
- 9: Prothesenspitze
- 10: neutrale Faser
- 11: proximaler Bereich des Schafts
- 12: distaler Bereich des Schafts
- 13: Prothesenschulter
- 14: Konus
- **α**: Steigungswinkel der medianen Flanke
- **β**: Steigungswinkel der lateralen Flanke

## Patentansprüche

1. Kurzschaftprothese für ein künstliches Hüftgelenk aufweisend einen implantierbaren Schaft (8), wobei der Schaft (8) einen distalen Bereich (12) und einen proximalen Bereich (11) umfasst, wobei der distale Bereich (12) wenigstens 50% der Gesamtlänge des Schafts (8) umfasst, weiterhin umfasst die Kurzschaftprothese eine mit dem Schaft (8) verbundene Prothesenschulter (13), welche so ausgebildet ist, dass die Prothesenschulter mit einem Konus (14) zur Aufnahme eines Prothesenkopfes (1) verbindbar ist, **dadurch gekennzeichnet, dass** der Schaft (8) eine mediale Flanke (2), eine laterale Flanke (6) und einen inneren Steg (5) aufweist, der beide Flanken (2,6) verbindet, wobei das Breitenverhältnis des Steges (5) zu den Flanken (2,6) im proximalen Bereich (11) des Schaftes bei mindestens 0,8 liegt, und das Tiefenverhältnis des Steges (5) zu den Flanken (2,6) im proximalen Bereich (11) des Schaftes (8) bei medial minimal 2,5 und lateral minimal 2 liegt, wodurch der Schaft (8) einen Doppel-T-Trägerquerschnitt im proximalen Bereich (11) aufweist.

2. Kurzschaftprothese gemäß Anspruch 1 **dadurch gekennzeichnet, dass** der Schaft (8) eine Titan- und/oder Titanoxid-Legierung umfasst.

3. Kurzschaftprothese gemäß Anspruch 1 **dadurch gekennzeichnet, dass** der Schaft (8) eine Cobalt-Chrom-Molybdän-Legierung umfasst.

4. Kurzschaftprothese gemäß einem der Ansprüche 1 oder 2 **dadurch gekennzeichnet, dass** der Schaft (8) wenigstens im proximalen Schaftbereich (11) eine biokompatible Beschichtung (3) aufweist, sodass die erfindungsgemäße Kurzschaftprothese zementfrei implantiert werden kann.

5. Kurzschaftprothese gemäß Anspruch 4 **dadurch gekennzeichnet, dass** die Beschichtung (3) Dicalciumphosphat oder Hydroxylapathit umfasst.

6. Kurzschaftprothese gemäß einem der vorigen Ansprüche **dadurch gekennzeichnet dass** der Schaft (8) wenigstens im proximalen Schaftbereich (11) eine Beschichtung (3) aus Reintitan aufweist.

7. Kurzschaftprothese gemäß einem der vorigen Ansprüche dadurchgekennzeichnet, dass die mediale Flanke (2) einen Steigungswinkel **α** von wenigstens 3° aufweist und die laterale Flanke (6) Steigungswinkel **β** von wenigstens 3,2° aufweist und beide Flanken im distalen Bereich auf den Schaftsteg (5) auslaufen.

8. Kurzschaftprothese gemäß einem der vorigen Ansprüche **dadurch gekennzeichnet, dass** sich die Steigungswinkel **α** und **β** voneinander unterscheiden.

## Claims

1. Short-stem prosthesis for an artificial hip joint comprising an implantable stem (8), the stem (8) comprising a distal portion (12) and a proximal portion (11), the distal portion (12) comprising at least 50% of the total length of the stem (8), furthermore, the short- stem prosthesis comprises a prosthesis shoulder (13) which is connected to the stem (8) and which is designed in such a way that the prosthesis shoulder can be connected to a cone (14) for receiving a prosthesis head (1), **characterised in that** in that the stem (8) has a medial flank (2), a lateral flank (6) and an inner bar (5) which connects the two flanks (2, 6), the width ratio of the bar (5) to the flanks (2, 6) in the proximal region (11) of the shaft being at least 0, 8, and the depth ratio of the bar (5) to the flanks (2, 6) in the proximal region (11) of the stem (8) is at a minimum of 2.5 medially and 2 laterally, as a result of which the stem (8) has a double-T beam cross-section in the proximal region (11).

2. Short- stem prosthesis according to claim 1, **characterised in that** the stem (8) comprises a titanium and/or titanium oxide alloy.

3. Short- stem prosthesis according to claim 1, **characterised in that** the stem (8) comprises a cobalt-chromium-molybdenum alloy.

4. Short- stem prosthesis according to one of claims 1 or 2, **characterised in that** the stem (8) has a biocompatible coating (3) at least in the proximal stem region (11), so that the short stem prosthesis according to the invention can be implanted without cement.

5. Short- stem prosthesis according to claim 4, **characterised in that** the coating (3) comprises dicalcium phosphate or hydroxylapathite.

6. Short- stem prosthesis according to one of the previous claims, **characterised in that** the stem (8) has a coating (3) of pure titanium at least in the proximal stem region (11).

7. Short- stem prosthesis according to one of the previous claims, **characterised in that** the medial flank (2) has an angle of inclination α of at least 3° and the lateral flank (6) has an angle of inclination β of at least 3.2° and both flanks end in the distal region on the stem bar (5).

8. Short stem prosthesis according to one of the previous claims, **characterised in that** the inclination angles α and β differ from each other.

## Revendications

1. Prothèse à manche courte pour une articulation de hanche artificielle, comprenant une manche implantable (8), la manche (8) comprenant une partie distale (12) et une partie proximale (11), la partie distale (12) comprenant au moins 50% de la longueur totale de la manche (8), en outre, la prothèse à manche courte comprend un épaulement de prothèse (13) relié à la manche (8), qui est conçu de telle sorte que l'épaulement de prothèse peut être relié à un cône (14) destiné à recevoir une tête de prothèse (1), **caractérisée en ce que** l'épaulement de prothèse (13) est conçu de telle sorte que la tête de prothèse (1) est reliée à la manche (8), que la manche (8) présente un flanc médial (2), un flanc latéral (6) et une barrette intérieure (5) qui relie les deux flancs (2, 6), le rapport de largeur de la barrette (5) aux flancs (2, 6) dans la zone proximale (11) de la manche étant d'au moins 0, 8, et le rapport de profondeur entre la barre (5) et les flancs (2, 6) dans la zone proximale (11) de la manche (8) est d'au moins 2,5 dans le sens médian et d'au moins 2 dans le sens latéral, la manche (8) présentant ainsi une section transversale de support en double T dans la zone proximale (11).

2. Prothèse à manche courte selon la revendication 1, **caractérisée en ce que** la manche (8) comprend un alliage de titane et/ou d'oxyde de titane.

3. Prothèse à manche courte selon la revendication 1, **caractérisée en ce que** la manche (8) comprend un alliage de cobalt-chrome-molybdène.

4. Prothèse à manche courte selon l'une des revendications 1 ou 2, **caractérisée en ce que** la manche (8) comporte, au moins dans la zone proximale (11) de la manche, un revêtement biocompatible (3), de sorte que la prothèse à manche courte selon l'invention peut être implantée sans ciment.

5. Prothèse à manche courte selon la revendication 4, **caractérisée en ce que** le revêtement (3) comprend du phosphate dicalcique ou de l'hydroxyapathite.

6. Prothèse à manche courte selon l'une des revendications précédentes, **caractérisée en ce que** la manche (8) présente, au moins dans la zone proximale (11) de la manche, un revêtement (3) en titane pur.

7. Prothèse à manche courte selon l'une des revendications précédentes, **caractérisée en ce que** le flanc médial (2) présente un angle d'inclinaison α d'au moins 3° et le flanc latéral (6) présente un angle d'inclinaison β d'au moins 3,2° et les deux flancs se terminent dans la zone distale sur la traverse sde manche (5).

8. Prothèse à manche courte selon l'une des revendications précédentes, **caractérisée en ce que** les angles d'inclinaison α et β sont différents l'un de l'autre.
